(19) Europäisches Patentamt
European Patent Office
Office européen des brevets

(11) Veröffentlichungsnummer: **0 087 704**
**B1**

(12) # EUROPÄISCHE PATENTSCHRIFT

(45) Veröffentlichungstag der Patentschrift: **02.10.85**

(51) Int. Cl.⁴: **C 07 C 145/04**, A 01 N 47/34

(21) Anmeldenummer: **83101618.3**

(22) Anmeldetag: **21.02.83**

(54) **N-sulfenylierte Harnstoffe, ein Verfahren zu ihrer Herstellung, diese enthaltende mikrobizide Mittel und ihre Verwendung.**

(30) Priorität: **02.03.82 DE 3207474**

(43) Veröffentlichungstag der Anmeldung:
**07.09.83 Patentblatt 83/36**

(45) Bekanntmachung des Hinweises auf die Patenterteilung:
**02.10.85 Patentblatt 85/40**

(84) Benannte Vertragsstaaten:
**CH DE FR GB IT LI NL SE**

(56) Entgegenhaltungen:
**EP - A - 0 046 557**
**FR - A - 1 493 603**
**FR - A - 2 341 567**
**US - A - 3 496 208**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Kühle, Engelbert, Dr., Bodelschwinghstrasse 42, D-5060 Bergisch Gladbach (DE)**
Erfinder: **Paulus, Wilfried, Dr., Deswatinesstrasse 90, D-4150 Krefeld 1 (DE)**
Erfinder: **Genth, Hermann, Dr., Heckerhof 60, D-4150 Krefeld (DE)**
Erfinder: **Brandes, Wilhelm, Dr., Eichendorffstrasse 3, D-5623 Leichlingen (DE)**
Erfinder: **Reinecke, Paul, Dr., Lessingstrasse 11, D-5090 Leverkusen 3 (DE)**

**EP 0 087 704 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue N-sulfenylierte Harnstoffe, ein Verfahren zu ihrer Herstellung, mikrobizide Mittel, die diese Verbindungen enthalten, und ihre Verwendung als Mikrobizide, im besonderen zum Schutz technischer Materialien und im Pflanzenschutz.

Die Verwendung einiger N-(Trihalogenmethylthio)-Verbindungen zum Schutz technischer Materialien gegen mikrobiellen Abbau ist bekannt (US 2 563 770; Journ. Agr. Food Chem. 14, 365 [1966]; Fette, Seifen, Anstrichmittel 68, 272 [1966]). Sie befriedigen jedoch nicht immer, da nicht alle Mikroorganismen von ihnen erfaßt werden; außerdem sind sie in Anstrich- und Imprägniermittel schlecht löslich.

Es wurden neue N-sulfenylierte Harnstoffe der Formel (I)

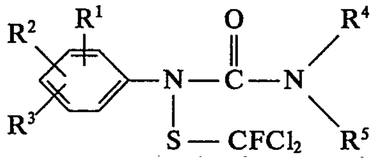

(I)

in der

$R^1$ bis $R^3$ gleich oder verschieden sind und Wasserstoff, Halogen, Nitro, Cyano, Niederalkyl, Niederalkoxy, Niederalkylmercapto, Trihalogenmethyl, Trihalogenmethoxy, Trihalogenmethylmercapto bedeuteten, wobei wenigstens einer der Reste $R^1$ bis $R^3$ von Wasserstoff verschieden ist, und

$R^4$ und $R^5$ gleich oder verschieden sind und für Wasserstoff, einen gegebenenfalls durch Halogen, Cyano, Nitro, Alkoxy, Alkylmercapto oder Dialkylamino substituierten Alkyl-, Alkenyl-, Alkinyl-, Cycloalkylrest oder gemeinsam mit dem Stickstoffatom für einen gegebenenfalls durch Sauerstoff, Schwefel oder Stickstoff unterbrochenes, 5- oder 6gliedriges Ringsystem stehen,

gefunden.

Die neuen N-sulfenylierten Harnstoffe haben eine hervorragende mikrobizide Wirkung und eignen sich besonders zum Schutz technischer Materialien und im Pflanzenschutz.

Halogen bedeutet im allgemeinen erfindungsgemäß Fluor, Chlor, Brom und Jod, bevorzugt Fluor und Chlor.

Alkyl bedeutet im allgemeinen erfindungsgemäß einen geradkettigen oder verzweigten Kohlenwasserstoffrest mit 1 bis 12, bevorzugt 1 bis 8, insbesondere bevorzugt 1 bis etwa 6 (Niederalkyl-)Kohlenstoffatomen. Beispielsweise seien die folgenden Alkylreste genannt: Methyl, Ethyl, Propyl, Isopropyl, Butyl, Isobutyl, Pentyl, Isopentyl, Hexyl und Isohexyl.

Alkoxy bedeutet im allgemeinen erfindungsgemäß einen an Sauerstoff gebundenen, geradkettigen oder verzweigten Kohlenwasserstoffrest mit 1 bis 12, bevorzugt 1 bis 8, insbesondere bevorzugt 1 bis etwa 6 (Niederalkoxy-)Kohlenstoffatomen. Beispielsweise seien die folgenden Alkoxyreste genannt: Methoxy, Ethoxy, Propoxy, Isopropoxy, Butoxy, Isobutoxy, Pentoxy, Isopentoxy, Hexoxy und Isohexoxy.

Alkylmercapto bedeutet im allgemeinen erfindungsgemäß einen an Schwefel gebundenen, geradkettigen oder verzweigten Kohlenwasserstoffrest mit 1 bis 18, bevorzugt 1 bis 8, insbesondere 1 bis etwa 6 (Niederalkylmercapto-)Kohlenstoffatomen. Beispielsweise seien die folgenden Alkylmercaptoreste genannt: Methylmercapto, Ethylmercapto, Propylmercapto, Isopropylmercapto, Butylmercapto, Isobutylmercapto, Pentylmercapto, Isopentylmercapto, Hexylmercapto und Isohexylmercapto.

Trihalogenmethyl bedeutet im allgemeinen erfindungsgemäß einen durch drei gleiche oder verschiedene Halogenatome substituierten Methylrest. Halogen bedeutet hier im allgemeinen Fluor, Chlor, Brom oder Jod, bevorzugt Fluor oder Chlor.

Trihalogenmethoxy bedeutet im allgemeinen erfindungsgemäß einen durch drei Halogenatome substituierten Methoxyrest. Halogen bedeutet hier im allgemeinen erfindungsgemäß Fluor, Chlor, Brom oder Jod.

Trihalogenmethylmercapto bedeutet im allgemeinen erfindungsgemäß einen durch drei Halogenatome substituierten Methylmercaptorest. Halogen bedeutet hier im allgemeinen Fluor, Chlor, Brom oder Jod, bevorzugt Fluor oder Chlor.

Alkenyl bedeutet im allgemeinen erfindungsgemäß einen geradkettigen oder verzweigten Kohlenwasserstoffrest mit 2 bis 12, bevorzugt 2 bis 8, insbesondere bevorzugt 2 bis etwa 6 (Niederalkenyl-)Kohlenstoffatomen mit einer oder mehreren, bevorzugt einer oder zwei Zweifachbindungen. Beispielsweise seien die folgenden Alkenylreste genannt: Allyl, Pentenyl und Octenyl.

Alkinyl bedeutet im allgemeinen erfindungsgemäß einen geradkettigen oder verzweigten Kohlenwasserstoffrest mit 2 bis 12, bevorzugt 2 bis 8, insbesondere bevorzugt 2 bis etwa 6 (Niederalkinyl-)Kohlenstoffatomen mit im wesentlichen einer Dreifachbindung. Beispielsweise seien hier die

Propin- und Butinreste genannt.

Cycloalkylreste bedeuten im allgemeinen erfindungsgemäß cyclische gesättigte Kohlenwasserstoffreste mit 4 bis 8, bevorzugt 5 und 6 Kohlenstoffatomen. Beispielsweise seien die folgenden Cycloalkylreste genannt: Cyclobutyl, Cyclopentyl, Cyclohexyl, Cycloheptyl, Cyclooctyl.

Die Alkyl-, Alkenyl-, Alkinyl- und Cycloalkylreste $R^4$ und $R^5$ können gegebenenfalls durch weitere Reste substituiert sein, die sich unter den Reaktionsbedingungen nicht verändern. Beispielsweise seien die folgenden Substituenten genannt: O-Alkyl, S-Alkyl, N-Dialkylamino, Nitro und Cyano.

Gemeinsam mit den Stickstoffatomen können die Reste $R^4$ und $R^5$ ein 5- oder 6gliedriges, gegebenenfalls durch ein weiteres Heteroatom unterbrochenes Ringsystem bilden. Als weitere Heteroatome seien O, S und N genannt. Beispielsweise seien hier folgende Reste genannt: Pyrrolidyl, Piperidyl, Morpholyl, Thiomorpholyl, Piperazyl.

Beispielsweise seien die folgenden N-sulfenylierten Harnstoffe genannt: N-(Fluordichlormethansulfenyl)derivate des N-Phenyl-N'-(methyl)-harnstoffes, N-Phenyl-N'-dimethyl-harnstoffes, N-Phenyl-N'-methyl-N-methoxymethyl-harnstoffes, N-4-Chlorphenyl-N'-(2-chlorethyl)-harnstoffes, N-3-Trifluormethylphenyl-N'-cyclohexyl-harnstoffes, N-3,4-Dichlorphenyl-N'-6-cyano-hexyl-harnstoffes, N-(4-Isopropylphenyl)-N'-pyrolidyl-harnstoffes, N-4-Toluidyl-harnstoffes, N-3-Anisidyl-N'-diallyl-harnstoffes.

Es wurde auch ein Verfahren zur Herstellung von N-sulfenylierten Harnstoffen gefunden, das dadurch gekennzeichnet ist, daß man N-substituierte Carbamidsäurefluoride der Formel (II)

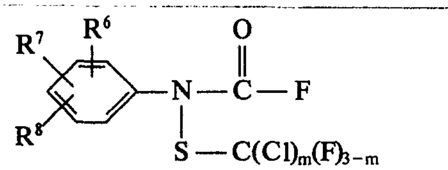

(II)

in der

$R^6$ bis $R^8$ gleich oder verschieden sind und Wasserstoff, Halogen, Nitro, Cyano, Alkyl, Alkoxy, Alkylmercapto, Trihalogenmethyl, Trihalogenmethoxy oder Trihalogenmethylmercapto bedeuten und

m für eine der Zahlen 0, 1, 2 oder 3 steht;

mit einem Amin der Formel (III)

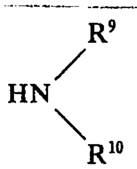

(III)

in der

$R^9$ und $R^{10}$ gleich oder verschieden sind und für Wasserstoff, einen gegebenenfalls substituierten Alkyl-, Alkenyl-, Alkinyl- oder Cycloalkylrest stehen oder gemeinsam mit dem Stickstoffatom ein 5- oder 6gliedriges, gegebenenfalls durch ein Heteroatom unterbrochenes Ringsystem bilden

in Gegenwart eines Verdünnungsmittels und eines säurebindenden Mittels umsetzt.

Das erfindungsgemäße Verfahren kann durch das folgende Formelbild erläutert werden:

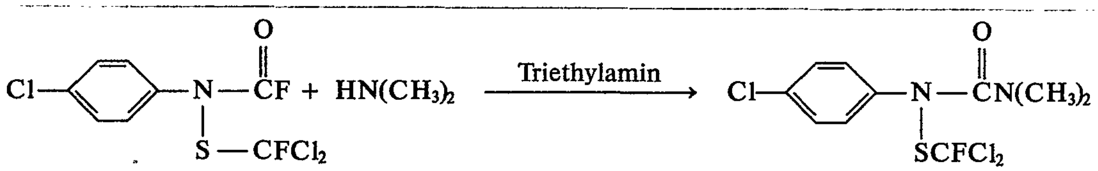


Die N-sulfenylierten Carbamidsäurefluoride für das erfindungsgemäße Verfahren sind an sich bekannt (DE-AS 1 297 095) und können aus Arylcarbamidsäurefluoriden und Trihalogenmethansulfenylchlorid in Gegenwart eines säurebindenden Mittels hergestellt werden.

Als Beispiele seien die N-(Trichlormethansulfenyl)- bzw. N-(Fluordichlormethansulfenyl)-Carbamidsäurefluoride des Anilins, 2-Chlor-anilins, 3,4-Dichloranilins, 3-Nitroanilins, 4-Toluidins, 4-Isopropylanilins, 3-Chlor-4-methoxyanilins, 2-Chlor-4-methyl-mercaptoanilins, 2-Chlor-4-trifluormethylanilins, 4-Difluor-chlormethylanilins, 3-Chlor-4-trifluormethoxyanilins und 3-Trifluormethylmercaptoanilins genannt.

Die Amine der Formel (III) sind an sich bekannt und können u. a. in an sich bekannter Weise durch Alkylierung von Ammoniak hergestellt werden. Beispielsweise seien die folgenden Amine genannt: Methylamin, Ethylamin, Isopropylamin, Allylamin, 2-Methoxyethylamin, 2-Ethylmercaptoethylamin, Neopentylamin, Isooctylamin, 2-Chlorethylamin, 6-Cyanohexylamin, Dimethylamin, Diallylamin, Dibutylamin, Cyclopentylamin, Cyclohexylamin, Pyrrolidin, Piperidin, Morpholin, Thiomorpholin, Piperazin, 1,1-Dimethylpropin-2-yl-amin und 1,1-Diethylpropin-2-yl-amin.

Im allgemeinen setzt man für das erfindungsgemäße Verfahren 1 bis 2,2 Mol des Amins, bezogen auf 1 Mol des N-substituierten Carbamidsäurefluorids, ein.

Als Verdünnungsmittel für das erfindungsgemäße Verfahren seien beispielsweise genannt: Kohlenwasserstoffe wie Toluol oder Xylol, Chlorkohlenwasserstoffe wie Dichlormethan oder Chlorbenzol, Ether wie Dioxan, Ketone wie Aceton, Alkohole wie Methanol oder Wasser.

Als säurebindendes Mittel für das erfindungsgemäße Verfahren seien beispielsweise genannt: tertiäre Amine wie Triethylamin, Dimethylbenzylamin, Pyridin, anorganische Basen wie Natriumhydroxid, Natrium- bzw. Kaliumcarbonat oder -bicarbonat oder ein Überschuß des zu verwendenden Amins der Formel (III).

Die säurebindenden Mittel werden im allgemeinen in einer Menge von 1 bis 2 Mol, bevorzugt 1,0 bis 1,2 Mol, bezogen auf 1 Mol des N-substituierten Carbamidsäurefluorids, eingesetzt.

Die Reaktionstemperatur für das erfindungsgemäße Verfahren kann in einem großen Bereich variiert werden; im allgemeinen arbeitet man im Temperaturbereich von 0 bis 100° C, bevorzugt von 20 bis 50° C.

Es ist jedoch auch möglich, einen kleinen Unter- oder Überdruck zu verwenden.

Das erfindungsgemäße Verfahren kann beispielsweise wie folgt durchgeführt werden:

Das N-substituierte Carbamidsäurefluorid wird in einem Verdünnungsmittel gelöst. In diese Lösung gibt man das Amin und rührt bei der jeweiligen Reaktionstemperatur. Hierbei fällt das Reaktionsprodukt aus, das in üblicher Weise, beispielsweise durch Kristallisation, isoliert wird.

Die erfindungsgemäßen N-sulfenylierten Harnstoffe können als Wirkstoffe zur Bekämpfung von Mikroorganismen verwendet werden. Als Mikroorganismen seien Bakterien, Pilze, Hefen, Algen und schleimformende Mikroben genannt. Vorzugsweise wirken die erfindungsgemäßen Wirkstoffe gegen Pilze, insbesondere gegen holzverfärbende Pilze und holzzerstörende Pilze (Basidiomyceten) sowie gegen schleimformende Organismen.

Die erfindungsgemäßen N-sulfenylierten Harnstoffe können als Wirkstoffe zur Bekämpfung dieser Mikroorganismen, im besonderen in technischen Materialien und im Pflanzenschutz, verwendet werden.

Technische Materialien sind nichtlebende Materialien, die für die Verwendung in der Technik zubereitet worden sind. Beispielsweise können technische Materialien, die durch erfindungsgemäße Wirkstoffe vor einer mikrobiellen Veränderung und Zerstörung geschützt werden sollen, Klebstoffe, Leime, Papiere und Kartone, Textilien, Leder, Holz, Anstrichmittel, Baustoffe, Kautschuk und Kunststoffartikel, Kühlschmiermittel und andere Materialien sein, die durch Mikroorganismen zersetzt werden können. Im Rahmen der zu schützenden Materialien seien auch Teile von Produktionsanlagen, beispielsweise Kühlwasserkreisläufe, genannt, die durch Mikroorganismen beeinträchtigt werden können. Im Rahmen der vorliegenden Erfindung seien technische Materialien, vorzugsweise Anstrich- und Imprägniermittel für Holz, genannt.

Als Mikroorganismen, die einen Abbau oder eine Veränderung der technischen Materialien bewirken können, seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora cerebella,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila und
Staphylococcus, wie Staphylococcus aureus.

Je nach ihrem Anwendungsgebiet können die erfindungsgemäßen Substanzen in die üblichen Formulierungen überführt werden, wie Lösungen, Emulsionen, Suspensionen, Pulver, Pasten und Granulate.

Diese können in an sich bekannter Weise hergestellt werden, z. B. durch Vermischen der Wirkstoffe mit einem Streckmittel, das aus flüssigem Lösungsmittel und/oder festen Trägerstoffen, gegebenenfalls unter Verwendung von oberflächenaktiven Mitteln, wie Emulgatoren und/oder Dispergiermitteln besteht, wobei beispielsweise im Falle der Benutzung von Streckmitteln gegebenenfalls organische Lösungsmittel als Hilfslösungsmittel verwendet werden können.

Organische Lösungsmittel für die Wirkstoffe können beispielsweise Alkohole wie niedere aliphatische Alkohole, vorzugsweise Ethanol oder Isopropanol oder Benzylalkohol, Ketone wie Aceton oder Methylethylketon, flüssige Kohlenwasserstoffe wie Benzinfraktionen, chlorierte Wasserstoffe wie 1,2-Dichlorethan sein.

Die Anwendungskonzentrationen der erfindungsgemäßen Substanzen richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen neuen Wirkstoffe können auch in Mischung mit anderen bekannten Wirkstoffen vorliegen. Beispielsweise seien die folgenden Wirkstoffe genannt: Benzimidazolyl-methylcarbamate, Tetramethyl-thiuramdisulfid, Zn-Salze von Dialkyldithiocarbamatan, 2,3,5,6-Tetrachlorisophthalonitril, Thiazolylbenzimidazol, N-Cyclohexyl-N-methoxy-2,5-dimethyl-3-furamid, Mercaptobenzthiazol und Phenolderivate, wie 2-Phenyl-phenol, (2,2'-Dihydroxy-5,5'-dichlor)-diphenylmethan.

Die erfindungsgemäßen N-sulfenylierten Harnstoffe können auch als Pflanzenschutzmittel, insbesondere zur Bekämpfung von Pilzen, verwendet werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Eine besonders gute Möglichkeit entfalten sie gegen parasitäre Pilze auf oberirdischen Pflanzenteilen, wie z. B. gegen den Erreger des Apfelschorfs (Fusicladium dendriticum) und gegen den Erreger der Braunpelzigkeit an Weizen (Leptosphaeria nodorum). Sie zeigen ferner eine Wirksamkeit gegen Reiskrankheiten (Erreger, z. B. Pyricularia Oryzae, Pellicularia sasakii). Daneben sind sie gegen samenbürstige Krankheiten wirksam, wie z. B. gegen Weizensteinbrand (Tilletia caries).

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und -Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid; aliphatische Kohlenwasserstoffe, wie Cyclohexan oder Paraffine, z. B. Erdölfraktionen, Akohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimetylsulfoxyd sowie Wasser; mit verflüssigten, gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde, und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit; sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohl, Polyvinylacetat.

Es können Farbstoffe wie anorganische Pigmente, z. B. Eisenoxid, Titanoxid, Ferrocyanblau, und organische Farbstoffe, wie Alizarin-, Azo- und Metallphthalocyanin-Farbstoffe und Spurennährstoffe, wie Salze von Eisen, Mangan, Bor, Kupfer, Kobalt, Molybdän und Zink, verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate, angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g/kg Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

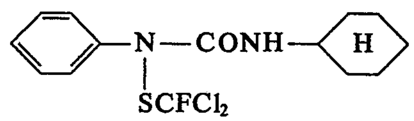

20 g (0,073 Mol) N-Phenyl-N-(fluordichlormethansulfenyl)-carbamidsäurefluorid werden in 200 ml Dioxan gelöst. In diese Lösung tropft man bei Raumtemperatur 14,6 g (1,475 Mol) Cyclohexylamin und läßt die Temperatur bis 38°C ansteigen. Man rührt etwa 30 Minuten lang und fällt das Reaktionsprodukt mit Wasser aus. Man saugt ab, wäscht mit Wasser und trocknet.
Ausbeute: 24 g = 93% der Theorie; Fp. 98—100°C.

### Beispiele 2 bis 17

In analoger Weise werden die folgenden N-substituierten Harnstoffe hergestellt:

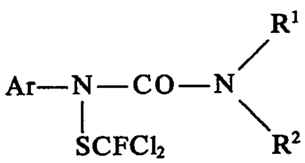

| Beispiel Nr. | Ar | $R^1$ | $R^2$ | Fp. ($n_D^{20}$) |
|---|---|---|---|---|
| 2 | $C_6H_5$ | $CH_3$ | $CH_3$ | 46— 50°C |
| 3 | 3,4-Cl-$C_6H_3$— | $CH_3$ | $CH_3$ | 100°C |
| 4 | 4-Cl-$C_6H_4$ | $CH_3$ | $CH_3$ | 86— 88°C |
| 5 | $C_6H_5$ | H | H | 141—142°C |
| 6 | 2-Cl$C_6H_4$ | H | H | 98—100°C |
| 7 | 4-Cl-$C_6H_4$ | H | H | 142—144°C |
| 8 | 3,5-Cl-$C_6H_3$ | H | H | 126—127°C |
| 9 | 3,5-Cl-$C_6H_3$ | $CH_3$ | H | 137—138°C |
| 10 | 3,5-Cl-$C_6H_3$ | $CH_3$ | $CH_3$ | 122—123°C |
| 11 | $C_6H_5$ | $CH_3$ | H | 132°C |

Fortsetzung

| Beispiel Nr. | Ar | $R^1$ | $R^2$ | Fp. ($n_D^{20}$) |
|---|---|---|---|---|
| 12 | 3-Cl-$C_6H_4$ | H | H | 95— 99°C |
| 13 | 3-Cl-$C_6H_4$ | $CH_3$ | H | 126—128°C |
| 14 | 3-Cl-$C_6H_4$ | $CH_3$ | $CH_3$ | (1,5681) |
| 15 | 4-Cl-$C_6H_4$ | $CH_3$ | H | 128—129°C |
| 16 | 3,4-Cl-$C_6H_3$ | H | H | 139—140°C |
| 17 | 3,4-Cl-$C_6H_3$ | $CH_3$ | H | 125—128°C |

Anwendungsbeispiele

Beispiel 18

Zum Nachweis der Wirksamkeit gegen Pilze werden die minimalen Hemm-Konzentrationen (MHK) von erfindungsgemäßen Wirkstoffen bestimmt.

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2wöchiger Lagerung bei 28°C und 60 bis 70% relativer Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle I angegeben.

Tabelle I

Angabe der MHK-Werte in mg/l bei der Einwirkung von N-sulfenylierten Harnstoffen folgender Formel auf Pilze:

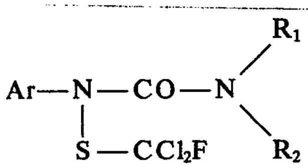

Tabelle I

| Substanz Ar | $R_1$ | $R_2$ | MHK in mg/l Alternaria tenuis | Aspergillus niger | Aureobasidium pullulans | Chaet. glob. | Coniophora puteana | Lentinus tigrinus | Penicil. glaucum | Polyporus versicolor | Sclerophona pityophila |
|---|---|---|---|---|---|---|---|---|---|---|---|
| $C_6H_5$ | H | H | 20 | 100 | 20 | 100 | 5 | 15 | 100 | 100 | 20 |
| 4-Cl-$C_6H_5$ | H | H | 3,5 | 10 | 5 | 35 | 0,5 | 1 | 50 | 15 | 10 |
| 3-Cl-$C_6H_5$ | H | H | 7,5 | 35 | 10 | 75 | 1 | 1 | 50 | 10 | 10 |
| 3,4-Cl-$C_6H_5$ | H | H | 1,5 | 10 | 10 | 15 | 0,5 | 1 | 20 | 2 | 10 |
| 3,5-Cl-$C_6H_5$ | H | H | 3,5 | 50 | 2 | 10 | 1,5 | 1 | 10 | 10 | 5 |
| $C_6H_5$ | H | $CH_3$ | 100 | 350 | 150 | 200 | 20 | 75 | 500 | 350 | 100 |
| 3-Cl-$C_6H_5$ | H | $CH_3$ | 7,5 | 10 | 10 | 20 | 0,5 | 1 | 200 | 20 | 10 |
| 4-Cl-$C_6H_5$ | H | $CH_3$ | 15 | 50 | 20 | 20 | 1 | 2 | 20 | 35 | 15 |
| 3,4-Cl-$C_6H_5$ | H | $CH_3$ | 1,5 | 10 | 5 | 10 | 0,3 | < 1 | 50 | 10 | 5 |
| 3,5-Cl-$C_6H_5$ | H | $CH_3$ | 5 | 35 | 2 | 35 | < 0,1 | 5 | 75 | 10 | 5 |
| 3-Cl-$C_6H_5$ | $CH_3$ | $CH_3$ | 5 | 20 | 5 | 10 | 0,3 | 1 | 200 | 20 | 10 |
| 3,4-Cl-$C_6H_5$ | $CH_3$ | $CH_3$ | 2 | 20 | 2 | 10 | 1,5 | < 1 | 50 | 10 | 15 |
| 3,5-Cl-$C_6H_5$ | $CH_3$ | $CH_3$ | 1 | 20 | 2 | 10 | < 0,1 | 1 | 150 | 750 | 1 |

Beispiel 19

(Wirkung gegen Schleimorganismen)

Verbindungen gemäß Tabelle II werden in Konzentrationen von jeweils 0,1 bis 100 mg/l in Allens Nährlösung (Arch. Mikrobiol. 17, 34 bis 53 [1952]), die in 4 l sterilem Wasser 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1% Caprolactam enthält, in wenig Aceton gelöst, zur Anwendung gebracht. Kurz vorher wird die Nährlösung mit Schleimorganismen (ca. $10^6$ Keime/ml), die aus bei der Polyamidherstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3wöchiger Kultur bei Raumtempratur noch völlig klar, d. h., die in wirkstofffreien Nährlösungen nach 3 bis 4 Tagen bemerkbare starke Vermehrung der Mikroben und Schleimbildung unterbleibt.

Für erfindungsgemäße Verbindungen folgender Formel werden auf diese Weise die MHK-Werte in Tabelle II ermittelt.

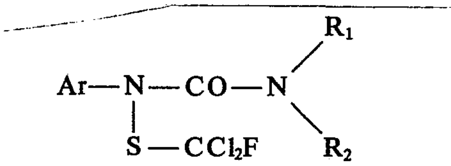

Tabelle II

| Substanz Ar | $R_1$ | $R_2$ | MHK in mg/l |
|---|---|---|---|
| 3,5-Cl-$C_6H_5$ | H | H | 2 |
| 3,5-Cl-$C_6H_5$ | H | $CH_3$ | 50 |
| 3,5-Cl-$C_6H_5$ | $CH_3$ | $CH_3$ | 2 |

Beispiel 20

Tilletia caries-Test (Weizen)/Saatgutbehandlung

Die Anwendung der Wirkstoffe erfolgt als Trockenbeizmittel. Sie werden zubereitet durch Abstrekken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut, das vorher mit 5 g Chlamydosporen von Tilletia caries pro kg Saatgut kontaminiert worden ist, 3 Minuten lang mit dem Beizmittel in einer verschlossenen Glasflasche.

Das Saatgut setzt man auf feuchtem Lehm unter einer Deckschicht aus einer Lage Mull und 2 cm feuchtem Vermiculit im Kühlschrank 10 Tage bei 10° C optimalen Keimungsbedingungen für die Sporen aus.

10 Tage nach der Aussaat erfolgt die Auswertung auf Sporenkeimung auf den Weizenkörnern.

Das Ergebnis des Testes ist in Vergleich zu dem handelsüblichen Zineb in Tabelle III angeführt:

Tabelle III
Tilletia caries-Test (Weizen)/Saatgutbehandlung

| Wirkstoff | Wirkstoffaufwandmenge in mg/kg Saatgut | Sporenkeimung in % der unbehandelten Kontrolle |
|---|---|---|
| Zineb (zum Vergleich) 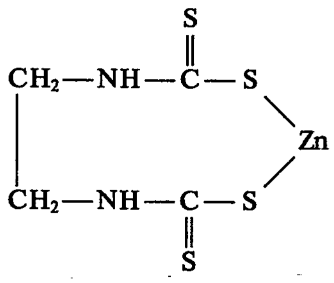 | 100 | 5,0 |
| erfindungsgemäß: | | |
| 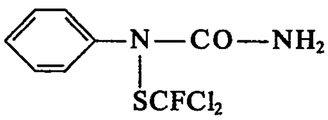 | 100 | 0,0 |
| 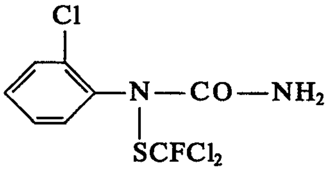 | 100 | 0,0 |
| 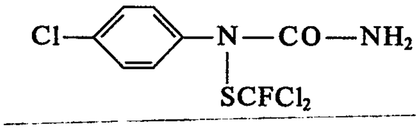 | 100 | 0,05 |

## Beispiel 21

### Leptosphaeria nodorum-Test (Weizen)/protektiv

Lösungsmittel: 100 Gew.-Teile Dimethylformamid
Emulgator: 0,25 Gew.-Teil Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptophaeria nodorum besprüht. Die Pflanzen verbleiben 48 Stunden bei 20°C und 100% rel. Luftfeuchtigkeit in einer Inkubationskabine.

Die Pflanzen werden in einem Gewächshaus bei einer Temperatur von ca. 15°C und einer rel. Luftfeuchtigkeit von ca. 80% aufgestellt.

10 Tage nach der Inokulation erfolgt die Auswertung.

Die deutliche Überlegenheit erfindungsgemäßer Wirkstoffe wird in der nachfolgenden Tabelle IV im Vergleich zu dem handelsüblichen Zineb angeführt:

Tabelle IV
Leptosphaeria nodorum-Test (Weizen)/protektiv

| Wirkstoff | Wirkstoffkonzentration in der Spritzbrühe in Gew.-% | Krankheitsbefall in % der unbehandelten Kontrolle |
|---|---|---|
| Zineb (zum Vergleich) 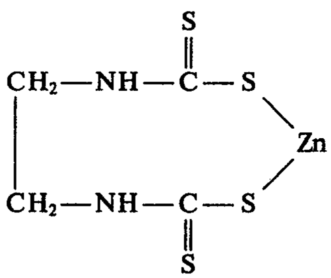 | 0,025 | 100 |
| erfindungsgemäß: | | |
| 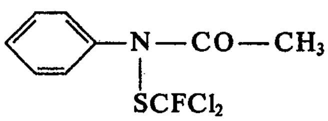 | 0,025 | 25,0 |
| 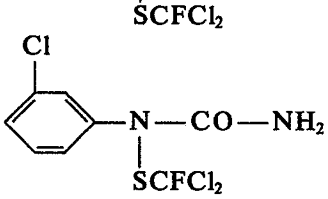 | 0,025 | 33,8 |
| 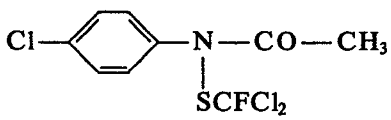 | 0,025 | 0,0 |

Beispiel 22

Venturia-Test (Apfel)/protektiv

Lösungsmittel: 4,7 Gew.-Teile Aceton
Emulgator: 0,3 Gew.-Teil Alkyl-Aryl-Polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100% rel. Luftfeuchtigkeit in einer Inkubationskabine.

Die Pflanzen werden dann im Gewächshaus bei 20° C und einer relativen Luftfeuchtigkeit von ca. 70% aufgestellt.

12 Tage nach der Inokulation erfolgt die Auswertung.

Die deutliche Überlegenheit der erfindungsgemäßen Wirkstoffe wird in der nachfolgenden Tabelle V im Vergleich zum Stand der Technik angeführt:

Tabelle V
Venturia-Test (Apfel)/protektiv

| Wirkstoff | Befall in % bei einer Wirkstoffkonzentration von 0,0005% |
|---|---|
| Zum Vergleich gemäß DE-OS 11 93 498 | |
| 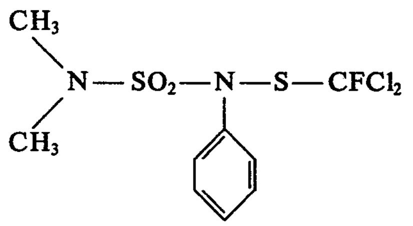 | 22 |
| erfindungsgemäß: | |
| 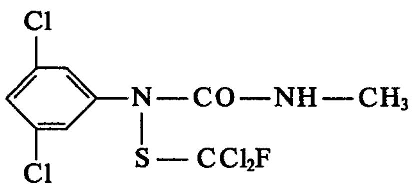 | 6 |
| 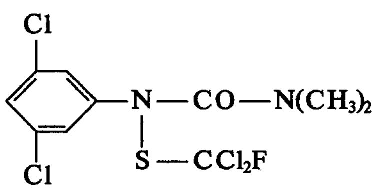 | 7 |

**Patentansprüche**

1. N-sulfenylierte Harnstoffe der Formel

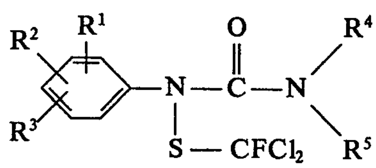

in der

$R^1$ bis $R^3$ gleich oder verschieden sind und Wasserstoff, Halogen, Nitro, Cyano, Niederalkyl, Niederalkoxy, Niederalkylmercapto, Trihalogenmethyl, Trihalogenmethoxy, Trihalogenmethylmercapto bedeuten, wobei wenigstens einer der Reste $R^1$ bis $R^3$ von Wasserstoff verschieden ist, und

$R^4$ und $R^5$ gleich oder verschieden sind und für Wasserstoff, einen gegebenenfalls durch Halogen, Cyano, Nitro, Alkoxy, Alkylmercapto oder Dialkylamino substituierten Alkyl-, Alkenyl-, Alkinyl-, Cycloalkylrest oder gemeinsam mit dem Stickstoffatom für einen gegebenenfalls durch Sauerstoff, Schwefel oder Stickstoff unterbrochenes, 5- oder 6gliedriges Ringsystem stehen.

2. Verfahren zur Herstellung von N-sulfenylierten Harnstoffen, dadurch gekennzeichnet, daß man N-substituierte Carbamidsäurefluoride der Formel

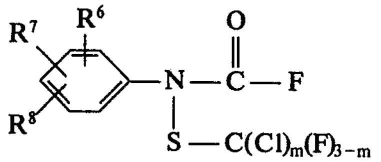

in der

$R^6$ bis $R^8$ gleich oder verschieden sind und Wasserstoff, Halogen, Nitro, Cyano, Alkyl, Alkoxy, Alkylmercapto, Trihalogenmethyl, Trihalogenmethoxy oder Trihalogenmethylmercapto bedeuten und

m für eine der Zahlen 0, 1, 2 oder 3 steht;

mit einem Amin der Formel

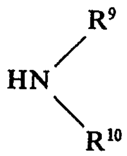

in der

$R^9$ und $R^{10}$ gleich oder verschieden sind und für Wasserstoff, einen gegebenenfalls substituierten Alkyl-, Alkenyl-, Alkinyl- oder Cycloalkylrest stehen oder gemeinsam mit dem Stickstoffatom ein 5- oder 6gliedriges, gegebenenfalls durch ein Heteroatom unterbrochenes Ringsystem bilden,

in Gegenwart eines Verdünnungsmittels und eines säurebindenden Mittels umsetzt.

3. Mikrobizides Mittel, enthaltend N-sulfenylierte Harnstoffe der Formel

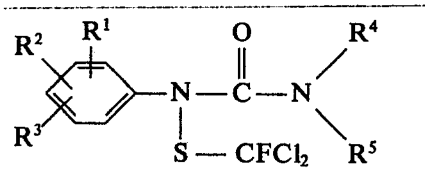

in der

$R^1$ bis $R^3$ gleich oder verschieden sind und Wasserstoff, Halogen, Nitro, Cyano, Niederalkyl, Niederalkoxy, Niederalkylmercapto, Trihalogenmethyl, Trihalogenmethoxy, Trihalogenmethylmercapto bedeuten, wobei wenigstens einer der Reste $R^1$ bis $R^3$ von Wasserstoff verschieden ist, und

$R^4$ und $R^5$ gleich oder verschieden sind und für Wasserstoff, einen gegebenenfalls durch Halogen, Cyano, Nitro, Alkoxy, Alkylmercapto oder Dialkylamino substituierten Alkyl-, Alkenyl-, Alkinyl-, Cycloalkylrest oder gemeinsam mit dem Stickstoffatom für einen gegebenenfalls durch Sauerstoff, Schwefel oder Stickstoff unterbrochenes, 5- oder 6gliedriges Ringsystem stehen.

4. Verwendung von mikrobiziden Mitteln nach Anspruch 3 zum Schutz technischer Materialien.

5. Verwendung von mikrobiziden Mitteln nach Anspruch 3 für den Pflanzenschutz.

6. Verwendung nach den Ansprüchen 4 und 5, dadurch gekennzeichnet, daß man N-sulfenylierte Harnstoffe nach Anspruch 1 mit Streckmitteln und/oder oberflächenaktiven Mitteln vermischt.

**Claims**

1. N-Sulphenylated ureas of the formula

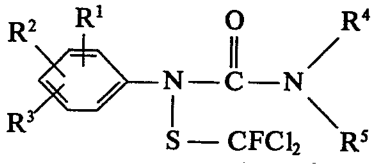

in which

$R^1$ to $R^3$ are identical or different and denote hydrogen, halogen, nitro, cyano, lower alkyl, lower alkoxy, lower alkylmercapto, trihalogenomethyl, trihalogenomethoxy or trihalogenomethylmercapto, at least one of the radicals $R^1$ to $R^3$ being different from hydrogen and

$R^4$ and $R^5$ are identical or different and represent hydrogen, an optionally halogen-, cyano-, nitro-, alkoxy-, alkylmercapto- or dialkylamino-substituted alkyl, alkenyl, alkinyl or cycloalkyl radical or, together with the nitrogen atom, represent a 5- or 6-membered ring system which is optionally interrupted by oxygen, sulphur or nitrogen.

2. Process for the preparation of N-sulphenylated ureas, characterised in that N-substituted carbamic acid fluorides of the formula

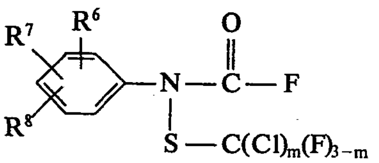

in which

$R^6$ to $R^8$ are identical of different and denote hydrogen, halogen, nitro, cyano, alkyl, alkoxy, alkylmercapto, trihalogenomethyl, trihalogenomethoxy or trihalogenomethylmercapto and

m represents one of the numbers 0, 1, 2 or 3;

are reacted with an amine of the formula

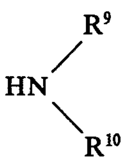

in which

$R^9$ and $R^{10}$ are identical or different and represent hydrogen, an optionally substituted alkyl, alkenyl, alkinyl or cycloalkyl radical, or, together with the nitrogen atom, form a 5- or 6-membered ring system which is optionally interrupted by a hetero atom,

in the presence of a diluent and an acid-binding agent.

3. Microbicidal agent containing N-sulpenylated ureas of the formula

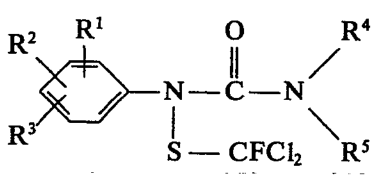

in which

$R^1$ to $R^3$ are identical or different and denote hydrogen, halogen, nitro, cyano, lower alkyl, lower alkoxy, lower alkylmercapto, trihalogenomethyl, trihalogenomethoxy or trihalogenomethylmercapto, at least one of the radicals $R^1$ to $R^3$ being different from hydrogen and

$R^4$ and $R^5$ are identical or different and represent hydrogen, an optionally halogen-, cyano-, nitro-, alkoxy-, alkylmercapto- or dialkylamino-substituted alkyl, alkenyl, alkinyl or cycloalkyl radical or, together with the nitrogen atom, represent a 5- or 6-membered ring system which is optionally interrupted by oxygen, sulphur or nitrogen.

4. Use of microbicidal agents according to Claim 3 for the protection of industrial materials.
5. Use of microbicidal agents according to Claim 3 for the protection of plants.
6. Use according to Claims 4 and 5, characterised in that N-sulphenylated ureas according to Claim 1 are mixed with extenders and/or surface-active agents.

**Revendications**

1. Urées N-sulfénylées de formule

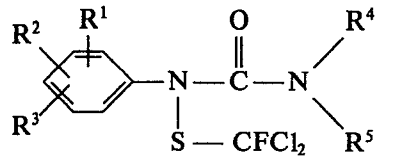

dans laquelle

$R^1$ à $R^3$ ayant des significations identiques ou différentes représentent chacun un atome d'hydrogène, d'halogène, un groupe nitro, cyano, alkyle inférieur, alcoxy inférieur, alkylmercapto inférieur, trihalogénométhyle, trihalogénométhoxy, trihalogénométhylmercapto, l'un au moins des symboles $R^1$ à $R^3$ ayant une signification autre que l'hydrogène, et

$R^4$ et $R^5$ sont identiques ou différents et représentent l'hydrogène, un groupe alkyle, alcényle, alcynyle, cycloalkyle éventuellement substitués par des halogènes, des groupes cyano, nitro, alcoxy, alkylmercapto ou dialkylamino, ou bien forment ensemble et avec l'atome d'azote un système cylique à 5 ou 6 chaînons éventuellement interrompu par l'oxygène, le soufre ou l'azote.

2. Procédé de préparation d'urées N-sulfénylées, caractérisé en ce que l'on fait réagir des fluorures d'acides carbamiques N-substitués de formule

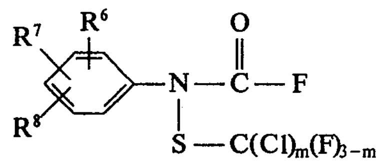

dans laquelle

$R^6$ à $R^8$ ayant des significations identiques ou différentes, représentent l'hydrogène, un halogène, un groupe nitro, cyano, alkyle, alcoxy, alkylmercapto, trihalogénométhyle, trihalogénométhoxy ou trihalogénométhylmercapto, et

m est égal à 0, 1, 2 ou 3,

avec une amine de formule

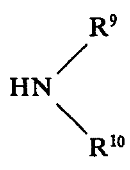

dans laquelle

$R^9$ et $R^{10}$ identiques ou différents, représentent l'hydrogène, un groupe alkyle, alcényle, alcynyle ou cycloalkyle éventuellement substitué ou forment ensemble et avec l'atome d'azote un système cyclique à 5 ou 6 chaînons éventuellement interrompu par un hétéroatome,

en présence d'un diluant et d'un agent fixant les acides.

3. Produit microbicide contenant des urées N-sulfénylées de formule

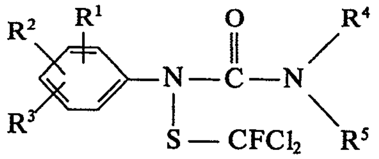

dans laquelle

| | |
|---|---|
| $R^1$ à $R^3$ | ayant des significations identiques ou différentes représentent chacun un atome d'hydrogène, d'halogène, un groupe nitro, cyano, alkyle inférieur, alcoxy inférieur, alkylmercapto inférieur, trihalogénométhyle, trihalogénométhoxy, trihalogénométhylmercapto, l'un au moins des symboles $R^1$ à $R^3$ ayant une signification autre que l'hydrogène, et |
| $R^4$ et $R^5$ | sont identiques ou différents et représentent l'hydrogène, un groupe alkyle, alcényle, alcynyle, cycloalkyle éventuellement substitués par des halogènes, des groupes cyano, nitro, alcoxy, alkylmercapto ou dialkylamino, ou bien forment ensemble et avec l'atome d'azote un système cyclique à 5 ou 6 chaînons éventuellement interrompu par l'oxygène, le soufre ou l'azote. |

4. Utilisation de produits microbicides selon la revendication 3 pour la protection des matériaux techniques.

5. Utilisation de produits microbicides selon la revendication 3 pour la protection des végétaux.

6. Utilisation selon les revendications 4 et 5, caractérisée en ce que l'on mélange des urées N-sulfénylées selon la revendication 1 avec des diluants et/ou des agents tensioactifs.